# EUROPÄISCHE PATENTSCHRIFT

(11) **EP 2 662 028 B1**
(45) Veröffentlichungstag und Bekanntmachung des Hinweises auf die Patenterteilung: **13.01.2016**
(21) Anmeldenummer: 13159567.0
(22) Anmeldetag: 15.03.2013
(51) Int. Cl.: A61B 10/00

(54) **Tupfer und Behälter zur Erfassung und sterilen Aufbewahrung von biologischen Proben**
Swab and container for taking biological samples and storing them in a sterile environment
Compresse et récipient destinés à la détection et au stockage stérile d'échantillons biologiques

(30) Priorität: 10.05.2012 DE 202012101720 U
(43) Veröffentlichungstag der Anmeldung: 13.11.2013
(73) Patentinhaber: Riess, Nathanael, 25436 Heidgraben (DE)
(72) Erfinder: Riess, Nathanael, 25436 Heidgraben (DE)
(74) Vertreter: Meyer, Ludgerus

(56) Entgegenhaltungen:
- EP-A1- 1 405 599
- DE-B3-102007 006 505
- DE-U1-202010 010 203
- US-A- 3 004 681
- US-A1- 2011 204 084

## Beschreibung

Die Erfindung betrifft einen Tupfer und Behälter zur Erfassung und sterilen Aufbewahrung von biologischen Proben, welche DNA-Material enthalten, nach dem Oberbegriff des Anspruchs 1.

Zur Spurensicherung an Tatorten werden heute zunehmend DNA-Proben genommen, die mit hoher Sicherheit eine Zuordnung der Probe zu einem Täter ermöglichen, wobei zur DNA-Analyse lediglich wenige Körperzellen ausreichend sind. Wenn eine geringe Zahl von Zellen vorhanden ist, ist es aber wichtig, dass die vorhandenen Spuren nicht noch durch Verunreinigungen oder Fremd-DNA-Spuren beeinträchtigt werden.

Eine große Schwachstelle bei einer forensischen Untersuchung sind offenbar die Testmaterialien selbst. So wurde ein Fall bekannt, in dem mit Fremd-DNA verunreinigte Wattestäbchen benutzt wurden, die die Ermittler auf eine falsche Fährte schickten. Obgleich die Herstellung derartiger Wattestäbchen in der Regel unter reinraumähnlichen Bedingungen steril erfolgt und sie damit frei von Mikroorganismen sind, kann gleichwohl Fremd-DNA-Material an das Wattestäbchen gelangen, wenn im Laufe des Herstellungsprozesses, insbesondere bei der Verpackung, nicht sorgfältig genug gearbeitet wurde oder wenn die Aufnahme der Probe unter erschwerten Bedingungen eines Tatorts zu erfolgen hat.

Es ist bekannt, Wattestäbchen durch radioaktive Bestrahlung steril zu machen. Es ist auch bekannt, die Produktion von Wattestäbchen dadurch zu verbessern, dass diese mit Ethylenoxid begast werden, wodurch DNA-Spuren deaktiviert werden. Die Begasung mit Ethylenoxid hat jedoch auch den Nachteil, dass später noch vorhandene Ethylenoxidgase die zu erfassenden DNA-Spuren zerstören können.

Aus der DE 199 57 861 A1 ist ein Probenbehälter zur Lagerung und Identifizierung von DNA/RNA-haltigen Material bekannt, bei dem der Probenbehälter eine starke hygroskopische Substanz enthält. Diese besteht z. B. aus Zeolith, Silikagel oder einem Molekularsieb, welches in einem getrennten Teil des Probenbehälters angeordnet ist. Durch die Aufteilung des Probenbehälters in einen Proberaum und mindestens einen Raum für die hygroskopische Substanz können Probe und hygroskopische Substanz getrennt verwendet werden. Die Verwendung der hygroskopischen Substanzen kann die Stabilität der zu analysierenden Probe verbessern. Das Problem der Verschmutzung des Probenbehälters selbst durch Fremd-DNA ist in dieser Druckschrift jedoch nicht angesprochen.

Aus der DE 60 2004 010 240 T2 ist ein Tupfer zur Aufnahme von biologischen Proben bekannt, der in einem Teströhrchen aufgenommen ist, wobei der Tupfer als Tupferstab ausgebildet ist, der an seiner Spitze einen Faserbausch aus Fasern mit hydrophilen Eigenschaften aufweist, um die Absorption von Proben zu ermöglichen, wobei die Faser an der Tupferspitze in der Form einer von durch Flockung abgelagerten Schicht hergestellt ist. Auch hier ist das Problem der Erfassung von Fremd-DNA vernachlässigt.

Aus der DE 20 2010 010 203 U1 ist ein Tupfer zur Erfassung und sterilen Aufbewahrung von biologischen Proben bekannt, welche DNA-Material enthalten, wobei der Tupfer eine an einem Träger befestigte Aufnahmefläche zur Erfassung der Probe enthält, der nach der Erfassung der Probe in einen verschließbaren Behälter einsetzbar ist. Der Träger ist dabei als Schaumstoffzylinder ausgebildet, dessen erstes Ende in einen Stopfen eingesetzt ist, der auf den einseitig offenen röhrchenförmigen Behälter abdichtend aufsetzbar ist. Das zweite Ende des Kunststoffzylinders ist mit einer Aufnahmefläche zur Aufnahme der Probe versehen, wobei die Aufnahmefläche im unbenutzten Zustand des Tupfers mit einer Schutzfolie abgedeckt ist.

Obgleich ein derartiger Tupfer alle Ansprüche an einen steril verpackten Tupfer erfüllt, ist seine Herstellung und Bereitstellung sehr zeitaufwändig.

Der Erfindung liegt daher die Aufgabe zugrunde, einen verbesserten Tupfer zur Erfassung und sterilen Aufbewahrung von biologischen Proben, welche DNA-Material enthalten, anzugeben, bei dem die Aufnahmefläche bis zur Aufnahme der Probe so lange wie möglich geschützt bleibt und der einfach, kostengünstig und schnell herstellbar ist.

Diese Aufgabe wird durch die im Anspruch 1 angegebene Erfindung gelöst. Vorteilhafte Weiterbildungen der Erfindung sind in Unteransprüchen angegeben.

Die Erfindung geht aus von einem Tupfer der in der DE 20 2010 010 203 U1 angegebenen Art.

Erfindungsgemäß ist der Behälter aus Kunststoff gebildet, der in seiner zylindrischen Umfangsfläche wenigstens eine gasdurchlässige Bohrung enthält.

Die Verwendung wenigstens einer gasdurchlässigen Bohrung in der Wandung des Behälters erlaubt einen relativen schnellen Eintritt von Sterilisationsgas in den Behälter und auch die schnelle Entgasung innerhalb von wenigen Tagen nach Abschluss der Sterilisation. Ein Eindringen von DNA-Fremdmaterial ist ausgeschlossen, weil dies nur an Gegenständen haftet und nicht über einen Gasstrom befördert wird.

Der erfindungsgemäße Tupfer enthält eine Aufnahmefläche, die in einem Röhrchen im Wesentlichen gasdicht aufgenommen ist und die innerhalb des Röhrchens abgedeckt ist, so dass die Aufnahmefläche solange geschützt bleiben kann, bis die DNA-Aufnahme erfolgt. Damit ergibt sich eine höhere Sicherheit gegen Fremd-DNA-Einflüsse. Der verwendete Schaumstoffzylinder erlaubt es, eine Probe durch flexibles Andrücken auf eine Fläche aufzunehmen, wobei die Aufnahmefläche durch die Flexibilität des Schaumstoffs der Oberflächenkontur der abzutastenden Fläche folgen kann. Die verwendete Schutzfolie kann nach Entnahme des Probenhalters aus dem Aufnahmeröhrchen noch so lange auf der Aufnahmefläche verbleiben, bis die Aufnahme der DNA-Probe selbst erfolgen soll. Damit ist höchste Sicherheit gegen Kontamination gewährleistet. Um die Probe auch nach der Aufnahme der DNA weiter gegen Fremdeinflüsse zu sichern, kann die Abdeckfolie erneut auf die Aufnahmefläche aufgebracht werden, von der sie erst wieder im Labor kurz vor der Auswertung der Probe entfernt wird.

Die Schutzfolie kann eine silikonisierte Papierabdeckung sein, die durch Haftklebung mehrfach auf die Aufnahmefläche aufgelegt werden kann.

Die Aufnahmefläche besteht insbesondere aus doppelseitig klebenden Kunststoffpunkten, deren Klebkraft gegenüber dem Träger erheblich höher als gegenüber der silikonisierten Abdeckung ist. Damit wird vermieden, dass beim Abziehen der Abdeckung der Kunststoffpunkt vom Träger abgelöst wird.

Der erfindungsgemäße Tupfer ist insbesondere zylindrisch mit einem Griffende und einem Halteende gestaltet, wobei das Griffende abdichtend auf das offene Ende des Behälters aufsetzbar ist. Das Halteende ist hohlzylindrisch derart ausgebildet, dass der Träger unter Klemmkraft in das Halteende einsetzbar ist. Zur Erhöhung der Haltekraft des Trägers am Halteende kann der Träger auch in dem Halteende festgeklebt sein.

Der röhrchenförmige Behälter zur Aufnahme des Tupfers ist insbesondere als durchsichtiges oder durchscheinendes Röhrchen ausgebildet. Auf der Außenseite des Röhrchens können sich Beschriftungsfelder befinden, die der Identifizierung der erfassten Probe dienen können.

Die Aufnahmefläche kann insgesamt eben sein oder auch dachförmig mit zwei Aufnahmeflächen, die z. B. zwei Proben aufnehmen können.

Die wenigstens eine Bohrung in der Wandung des Behälters befindet sich in einem Bereich des Behälters, in dem sich der in den Behälter eingesetzte Träger gegenüber der Innenwandung des Behälters befindet. Diese Ausbildung sorgt dafür, dass der freie Gasdurchstrom durch die eine oder mehrere Bohrungen eingeschränkt wird, insbesondere dann, wenn der Träger bzw. Schaumstoffzylinder die Innenwandung des Behälters kontaktiert.

In einer weiterbildenden Ausführungsform kann auch vorgesehen sein, den Träger bzw. den Stopfen mit einem integrierten RFID-Transponder zu versehen, um ggf. eine automatisierte Auswertung von Proben zu ermöglichen, durch die verhindert werden kann, dass im Labor eine manuelle Behandlung der mit DNA-Spuren versehenen Probe erforderlich ist.

Zur Herstellung des Tupfers mit steriler Aufnahmefläche zum Empfang von DNA-Material ist insbesondere vorgesehen, dass der Tupfer vor seiner Benutzung in einer Behandlungskammer sterilisiert und entgast wird. Die Sterilisierung erfolgt insbesondere durch Ethylenoxid, wobei der Tupfer nach der Sterilisierung so lange entgast wird, bis der Restwert des Gases den Wert 1 ppm unterschritten hat.

Auf diese Weise wird jegliches DNA-Fremdmaterial, das bei der Herstellung des Tupfers u. U. auf die Aufnahmefläche gelangt sein kann, zerstört, ohne dass nach Ablauf der Entgasungszeit noch Sterilisationsgas vorhanden ist, das eine neue aufzunehmende Probe zerstören könnte.

Die Erfindung wird nachstehend anhand eines Ausführungsbeispiels näher erläutert. Es zeigen:
- Fig. 1: einen Querschnitt durch einen erfindungsgemäßen Tupfer, der in ein Röhrchen eingesetzt ist,
- Fig. 2: eine vergrößerte Ansicht der Elemente der Aufnahmefläche, und
- Fig. 3: eine Abwandlung eines Tupfers von Fig. 1.

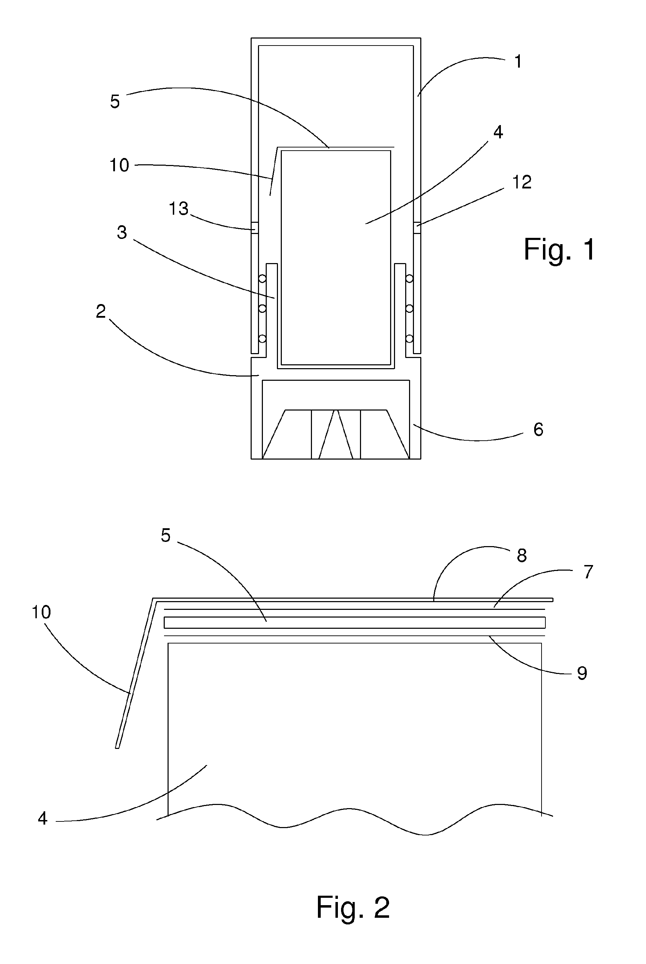

Fig. 1 zeigt ein hohlzylindrisches Kunststoffröhrchen 1, insbesondere aus Polystyrol, in das der aus Kunststoff, wie PVC oder Polyamid, vorzugsweise Polyethylen, hergestellte Stopfen 2 einsetzbar ist. Dieser weist auf seinem Stopfenrand umlaufende ringförmige Erhebungen zur Abdichtung gegenüber dem Behälter 1 auf.

Der Stopfen 2 enthält ein hinteres Griffende und eine vorderes Halteende 3, welches eine zylinderförmige Ausnehmung aufweist, in die ein weichelastischer Schaumstoffzylinder 4, vorzugsweise aus vernetztem LDPE einsetzbar ist. Dieser ist in dem Halteende 3 entweder eingeklemmt oder darin eingeklebt. Die Mantelfläche des Schaumstoffzylinders und die Innenwandung des Röhrchens weisen einen Abstand von 1 - 2 mm auf.

Am vorderen Ende des Schaumstoffzylinders 4 befindet sich eine auf die Stirnseite des Schaumstoffzylinders aufgeklebte Aufnahmefläche, deren Rückseite gegenüber dem Schaumstoffzylinder eine hohe Klebekraft aufweist und deren Vorderseite eine etwa um den Faktor 20 geringere Klebekraft aufweist. Auf die offene Vorderseite ist eine Schutzfolie aufgesetzt, die an der Aufnahmefläche 5 nur haftet und daher leicht von der Aufnahmefläche ablösbar ist. Zum Entfernen der Schutzfolie weist diese einen seitlichen Anfassstreifen 10 auf, mit dessen Hilfe die Schutzfolie von dem Schaumstoffkörper 4 entfernt werden kann.

Das Röhrchen 1 enthält zwei seitliche Bohrungen 12 und 13, die einen Innendurchmesser von etwa 0,8 mm aufweisen. Diese erlauben einen Zugriff und Abfluss von Sterilisiergas zum und aus dem Inneren des Röhrchens. Die Bohrungen befinden sich in einem Bereich gegenüber dem Schaumstoffzylinder 4.

Fig. 2 zeigt den Klebebereich mit Schaumstoffzylinder 4 und Aufnahmefläche 5 in vergrößerter Darstellung. Die Aufnahmefläche 5 weist auf ihrer Rückseite eine dünne Klebeschicht 9 und auf ihrer Vorderseite eine dünne Klebeschicht 7 auf. Die Aufnahmefläche 5 besteht insbesondere aus Polyesterfolie, die mit einem modifizierten Lösemittelacrylat am Schaumstoffkörper 4 festgeklebt ist. Die Klebeschicht 7 auf der Vorderseite ist ein Dispersionsreinacrylat, das schwach haftend ist und mit einer aus Silikonpapier bestehenden Schutzfolie 8 abgedeckt wird. Die Scherfestigkeiten zwischen der Klebeschicht 7 und der Klebeschicht 9 unterscheiden sich im Verhältnis von etwa 1:20.

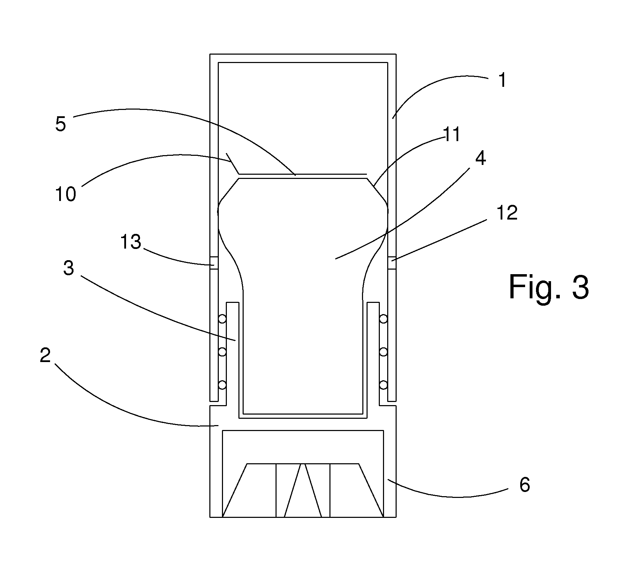

In der in Fig. 3 dargestellten Ausführungsform kontaktiert wenigstens ein vorderer Bereich des Schaumstoffkörpers 4 die Innenwandung des Röhrchens 1. Das durch die Bohrungen 12 und 13 ein- oder austretende Gas durchdringt den für diesen Fall gasdurchlässigen Schaumstoffzylinder. Zur Erleichterung der Einführung des Schaumstoffzylinders in das Röhrchen weist dieser eine Einführschräge 11 auf. Diese Ausführungsform verringert die Gasstromgeschwindigkeit durch die Bohrungen 12 und 13.

Die Herstellung des erfindungsgemäßen Tupfers erfolgt dadurch, dass der Schaumstoffkörper 4 zunächst in dem Halteende 3 befestigt wird, dass danach die Aufnahmefläche 5 zusammen mit der Schutzfolie 8 auf die Stirnseite des Schaumstoffkörpers 4 aufgebracht wird und der Stopfen mit eingesetztem Schaumstoffkörper 4 in das Röhrchen 1 eingesetzt wird. Mehrere derart hergestellter Röhrchen werden dann in eine Sterilisationsfolie gegeben, mittels Ethylenoxid sterilisiert, welches den gesamten Tupfer durchdringt, und danach so lange entgast, bis ein minimaler Restwert von 1 ppm Ethylenoxid erreicht ist. Dieser Wert ergibt sich nach etwa 1 - 2 Tagen Entgasungszeit. Danach können die Röhrchen für längere Zeit aufbewahrt werden, bis sie benötigt werden.

Zur Aufnahme einer Probe wird ein Röhrchen der Sterilisationsfolie entnommen und unmittelbar am Ort der Probe geöffnet. Dann wird die Schutzfolie 8 von der Aufnahmefläche 5 abgezogen und die auf der Vorderseite des Schaumstoffkörpers angebrachte Aufnahmefläche 5 wird auf die Stelle aufgedrückt, von der die Probe zu nehmen ist. Durch die Klebkraft der Aufnahmefläche haftet die Probe an der Aufnahmefläche. Die Abdeckung kann dann erneut auf den Klebepunkt aufgesetzt werden. Da hierbei jedoch die Gefahr einer Fremdkombination besteht, wird die Abdeckfolie vorzugsweise weggeworfen und der Stopfen 2 mit dem Schaumstoffkörper 4 unmittelbar in das Röhrchen 1 eingeführt. Das Röhrchen wird dann einem Labor zur Untersuchung zugeleitet.

Ein Röhrchen weist etwa eine Länge von 5 cm und einen Durchmesser von 2 cm auf.

### Bezugszeichen

- 1: Röhrchen
- 2: Stopfen
- 3: Halteende
- 4: Schaumstoffzylinder, Träger
- 5: Aufnahmefläche
- 6: Griffende
- 7: Klebeschicht
- 8: Schutzfolie
- 9: Klebeschicht
- 10: Anfassstreifen
- 11: Einführschräge
- 12: Bohrung
- 13: Bohrung

## Patentansprüche

1. Tupfer und Behälter (1) zur Erfassung und sterilen Aufbewahrung von biologischen Proben, welche DNA-Material enthalten, wobei der Tupfer eine an einem Träger (4) befestigte sterilisierte Aufnahmefläche (5) zur Erfassung der Probe enthält, und der Träger (4) aus dem verschließbaren Behälter (1) entnehmbar und nach Erfassung der Probe wieder in den Behälter (1) einsetzbar ist, wobei der Träger (4) als Schaumstoffzylinder ausgebildet ist, dessen erstes Ende in einen Stopfen (2) eingesetzt ist, der auf den einseitig offenen röhrchenförmig ausgebildeten Behälter (1) aufsetzbar ist und das zweite Ende des Schaumstoffzylinders stirnseitig mit einer an dem Schaumstoffzylinder angeordneten Aufnahmefläche (5) zur Aufnahme der Probe versehen ist, **dadurch gekennzeichnet, dass** der Behälter (1) aus Kunststoff gebildet ist, der in seiner zylindrischen Umfangsfläche wenigstens eine gasdurchlässige Bohrung enthält, dass die wenigstens eine Bohrung in einem Bereich des Behälters ausgebildet ist, in dem sich der in den Behälter eingesetzte Träger gegenüber der Innenwand des Behälters befindet, und dass der Träger die Innenwand des Behälters kontaktiert, wobei der Träger gasdurchlässig ausgebildet ist.

2. Tupfer und Behälter nach Anspruch 1, **dadurch gekennzeichnet, dass** der Behälter mehrere auf dem Umfang des Behälters verteilte Bohrungen enthält.

3. Tupfer und Behälter nach Anspruch2, **dadurch gekennzeichnet, dass** der Behälter zwei gegenüberliegende Bohrungen mit einem Innendurchmesser von jeweils 0,8 mm enthält.

4. Tupfer und Behälter nach Anspruch1, **dadurch gekennzeichnet, dass** der Träger an seiner in den Behälter einführbaren Stirnseite im Randbereich mit einer Einführschräge versehen ist.

5. Tupfer und Behälter nach Anspruch 1, **dadurch gekennzeichnet, dass** der Stopfen (2) zylindrisch mit einem Griffende (6) und einem Halteende (3) ausgestattet ist, wobei das Griffende (6) abdichtend auf das offene Ende des Behälters (1) aufsetzbar ist und das Halteende (3) hohlzylindrisch derart ausgebildet ist, dass der Träger (4) unter Klemm- oder Klebekraft in das Halteende (3) einsetzbar ist.

6. Tupfer und Behälter nach Anspruch 1, **dadurch gekennzeichnet, dass** die Aufnahmefläche (5) des Trägers im unbenutzten Zustand des Tupfers mit einer Schutzfolie (8) abgedeckt ist, und dass die Schutzfolie (8) eine silikonisierte Papierabdeckung mit einem Anfassstreifen (10) ist.

7. Tupfer und Behälter nach Anspruch 1, **dadurch gekennzeichnet, dass** der Stopfen (2) aus Kunststoff gebildet ist.

## Claims

1. Swab and container (1) for the collection and sterile storage of biological samples which contain DNA material, wherein the swab contains a sterile receiving surface (5) for collecting the sample, wherein said receiving surface is attached to a carrier (4) which can be removed from the closable container (1) and reintroduced therein after collecting the sample, wherein the carrier (4) is designed as a foam cylinder the first end of which is inserted into a stopper (2) that can be attached to the tube-shaped container (1) that is open on one side and the second end of said foam cylinder is frontally provided with a receiving surface (5) for receiving the sample, **characterized in that** the container (1) is made of plastic and that it contains, in its cylindrical circumference, at least one gas-permeable bore, **in that** the at least one bore is provided in a part of the container in. which the carrier inserted into the container is arranged opposite the inner wall of the container and **in that** the carrier is in contact with the internal wall of the container, while the carrier is gas-permeable.

2. Swab and container according to claim 1, **characterized in that** the container comprises several bores that are arranged on the circumference of the container.

3. Swab and container according to claim 2, **characterized in that** the container comprises two opposite bores with an internal diameter of respectively 0.8 mm.

4. Swab and container according to claim 1, **characterized in that** the carrier on its frontside, that is insertable into the container, is provided with an inclined introduction surface in its peripheral area.

5. Swab and container according to claim 1, **characterized in that** the stopper (2) is cylindrically provided with a handle end (6) and a grip end (3), whereas the handle end (6) is sealingly attachable to the open end of the container (1) and the holding end (3) is hollow-cylindrically designed in such a manner that the carrier (4) can be inserted into the grip end (3) under clamping or adhesive force.

6. Swab and container according to claim 1, **characterized in that** the receiving surface (5) of the carrier in unused condition of the swab is covered by a protective film (8) and that the protective film (8) is a siliconized paper cover with a grab strip (10).

7. Swab and container according to claim 1, **characterized in that** the stopper is made from plastic.

## Revendications

1. Tampon et récipient (1) pour le prélèvement et la conservation stérile d'échantillons biologiques qui contiennent un matériau ADN, étant précisé que le tampon contient une surface de réception stérilisée (5), fixée à un support (4), pour le prélèvement de l'échantillon, et que le support (4) est apte à être enlevé du récipient obturable (1) et à être remis dans ledit récipient (1) après prélèvement de l'échantillon, étant précisé que le support (4) est conçu comme un cylindre en mousse dont une première extrémité est introduite dans un bouchon (2) apte à être posé sur le récipient (1) de forme tubulaire et ouvert. d'un côté, et que la seconde extrémité du cylindre en mousse est pourvue, côté frontal, d'une surface de réception (5) disposée sur ledit cylindre en mousse et destinée à recevoir l'échantillon, **caractérisé en ce que** le récipient (1) est en matière plastique qui contient dans sa surface périphérique cylindrique au moins un perçage perméable aux gaz, **en ce que** le ou les perçages sont formés dans une zone du récipient dans laquelle le support introduit dans le récipient se trouve en face de la paroi intérieure dudit récipient, et **en ce que** le support touche la paroi intérieure du récipient, étant précisé que ledit support est perméable aux gaz.

2. Tampon et récipient selon la revendication 1, **caractérisé en ce que** le récipient contient plusieurs perçages répartis sur sa circonférence.

3. Tampon et récipient selon la revendication 2, **caractérisé en ce que** le récipient contient deux perçages opposés présentant chacun un diamètre intérieur de 0,8 mm.

4. Tampon et récipient selon la revendication 1, **caractérisé en ce que** le support est pourvu, sur son côté frontal apte à être introduit dans le récipient, dans la zone du bord, d'un biseau d'introduction.

5. Tampon et récipient selon la revendication 1, **caractérisé en ce que** le bouchon (2) a une forme cylindrique, avec une extrémité de préhension (6) et une extrémité de fixation (3), étant précisé que l'extrémité de préhension (6) est apte à être posée de manière étanche sur l'extrémité ouverte du récipient (1), et que l'extrémité de fixation (3) a une forme cylindrique creuse de telle sorte que le support (4) puisse être introduit dans ladite extrémité de fixation (3) sous l'action d'une force de serrage ou d'adhésion.

6. Tampon et récipient selon la revendication 1, **caractérisé en ce que** la surface de réception (5) du support, dans l'état non utilisé du tampon, est couverte par un film de protection (8), et **en ce que** le film de protection (8) est un recouvrement en papier siliconé, avec une bande à saisir (10).

7. Tampon et récipient selon la revendication 1, **caractérisé en ce que** le bouchon (2) est en matière plastique.
